# EUROPEAN PATENT APPLICATION

(11) **EP 2 226 019 A1**
(43) Date of publication of application: **08.09.2010**
(21) Application number: 08867697.8
(22) Date of filing: 24.12.2008
(51) Int. Cl.: A61B 17/06

(54) **MEDICAL SUTURE NEEDLE**

(30) Priority: 27.12.2007 JP 2007336238
(71) Applicant: MANI Inc., Utsunomiya Tochigi 321-3231 (JP)
(72) Inventor: MATSUTANI, Masaaki, Utsunomiya-shi Tochigi 321-3231 (JP); FUKUDA, Shouichi, Utsunomiya-shi Tochigi 321-3231 (JP); FUKUDA, Masatoshi, Utsunomiya-shi Tochigi 321-3231 (JP); AKUTSU, Shinichi, Utsunomiya-shi Tochigi 321-3231 (JP); KATO, Kazuaki, Utsunomiya-shi Tochigi 321-3231 (JP); YANO, Shinichi, Utsunomiya-shi Tochigi 321-3231 (JP)
(74) Representative: Göhring, Robert
(86) International application number: PCT/JP2008/073476
(87) International publication number: WO 2009/084549

(57) **Abstract**

The problem that a metal wire joined integrally to a hole formed in a proximal end face of a medical suture needle is susceptible to bending damage is reduced.

A suture needle A has a blind hole 5 which is formed in a proximal end face 3, inserts the end of a wire 10 thereinto, and joins the wire 10 thereto by caulking, and a counterbore 6 which is formed on the proximal end face 3 side of the blind hole 5 and has a dimension D at least in the proximal end face 3 larger than a dimension d of the blind hole 5 and a depth L smaller than a depth of a caulked portion with respect to the blind hole 5. A suture needle with wire inserts the end of the wire 10 into the blind hole 5 to caulk the outer circumference of the suture needle corresponding to the blind hole 5, thereby integrally joins the wire 10 thereto.

## Description

### Technical Field

The present invention relates to a medical suture needle which is advantageously used for suturing bones including breastbones.

### Background Art

When bones including breastbones are sutured, metal wires are used. The metal wire is joined integrally to the proximal end of a medical suture needle. The medical suture needle pierces through a bone, passes the metal wire therethrough, and joins the metal wire passing through the bone thereto to suture a desired point.

The metal wire is joined to the medical suture needle by forming a hole larger than the thickness of the metal wire in the proximal end face of the medical suture needle, inserting the end of the metal wire into the hole, and caulking the outer circumferential portion of the medical suture needle corresponding to the hole to reduce the diameter of the metal wire. When the joining strength of the metal wire integrated with the medical suture needle is low, the metal wire is fallen out of the hole of the medical suture needle at the time of a suturing operation, thereby interfering with the progress of the operation. For this reason, a technique of reliably joining both has been proposed.

For example, a technique described in Patent Document 1 relates to a method of manufacturing a breastbone suturing wire which is thin at both ends and is thick in its center. A hole having a diameter of 0.4 mm and a depth of 0.2 to 0.3 mm is formed in the end face of a thick wire having a diameter of about 0.8 mm, and a neck is formed to the side surface at the end of a thin wire having a diameter of 0.3 mm. The end of the thin wire is inserted into the hole of the thick wire. Therefore, a dice uniformly applies a force to the outer circumference of the thick wire for pressure bonding. Thus, the inside portion of the thick wire is engaged in the necked portion of the thin wire to secure tension strength.

[Patent Document 1] Japanese Patent Application Laid-Open No. 2002-078712

### Disclosure of the Invention

In the technique described in Patent Document 1, the thick wire and the thin wire can be strongly integrated. However, when the technique is directly applied to the joining of the medical suture needle and the metal wire, the problem that the metal wire is susceptible to bending damage from near the hole formed in the proximal end face of the medical suture needle arises.

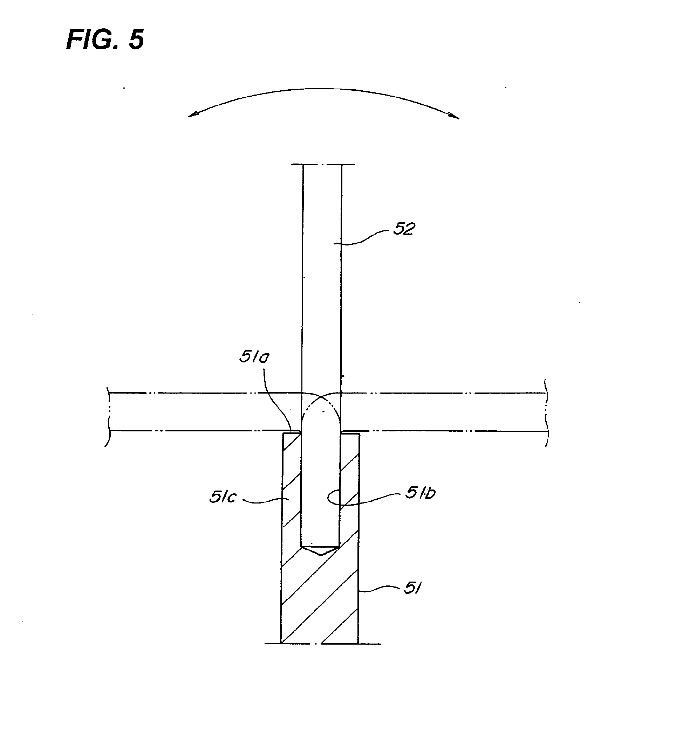

As shown in Fig. 5, the end of a wire 52 is inserted into a blind hole 51b formed in a proximal end face 51a of a suture needle 51, and a force is uniformly applied to the outer circumference of a proximal end 51c for caulking. Thereafter, the suture needle 51 is fixed by a vice to perform 180° reverse bending so that the wire 52 is 90° with respect to the proximal end face 51a. At this time, the wire 52 is bending damaged in one reciprocation or two reciprocations.

An object of the present invention is to provide a medical suture needle which can reduce the problem that a metal wire joined integrally to a hole formed in the proximal end face of the medical suture needle is susceptible to bending damage and a medical suture needle with metal wire.

To address the above problems, a medical suture needle according to the present invention which joins a metal wire to a proximal end face, having a blind hole which is formed in the proximal end face, inserts the end of the metal wire thereinto, and joins the metal wire thereto by caulking, and a counterbore which is formed on the proximal end face side of the blind hole and has a dimension at least in the proximal end face larger than a dimension of the blind hole and a depth smaller than a depth of a caulked portion with respect to the blind hole.

A medical suture needle with metal wire according to the present invention have a blind hole which is formed in a proximal end face and inserts the end of a metal wire thereinto, and a counterbore which is formed on the proximal end face side of the blind hole and has a dimension at least in the proximal end face larger than a dimension of the blind hole, wherein the end of the metal wire is inserted into the blind hole to caulk the outer circumference of the medical suture needle corresponding to the blind hole, thereby integrating the metal wire therewith.

In the medical suture needle (hereinafter, called "suture needle") according to the present invention, the blind hole for joining the metal wire (hereinafter, called "wire") thereto by caulking is formed in the proximal end face, and the counterbore having a depth smaller than a depth of the caulked portion with respect to the blind hole on the proximal end face side of the blind hole. When the end of the wire is inserted into the blind hole for caulking, a gap is formed between the counterbore and the wire because the counterbore has a dimension in the proximal end face larger than a dimension of the blind hole. Accordingly, the portion of the wire corresponding to the counterbore has the degree of freedom of bending in the range of the gap between the wire and the counterbore.

Therefore, the wire is bent starting from the caulked blind hole. However, the portion of the wire corresponding to the counterbore is contacted with the counterbore and is restricted so that the wire cannot be bent at a large curvature. A large stress cannot concentrate on the local portion of the wire, in particular, on the portion close to the proximal end face of the suture needle, e.g., near the boundary between the counterbore and the proximal end face. The problem that the wire is susceptible to bending damage can be improved.

In the suture needle with wire according to the present invention, the end of the wire is inserted into the blind hole formed in the proximal end face to caulk the outer circumference of the suture needle corresponding to the blind hole. Therefore, the degree of freedom of bending of the portion of the wire corresponding to the counterbore can be secured. Thus, the problem that the wire is susceptible to bending damage can be improved.

As described above, in the present invention, the susceptibleness to bending damage can be improved without changing the material and thickness of the wire. Reliable suturing can be realized using the related art wire in a bone suturing operation.

### Brief Description of the Drawings

Fig. 1 is a diagram of assistance in explaining the overall configuration of a suture needle;
Figs. 2(a) and 2(b) are cross-sectional views of a proximal end portion of the suture needle, which are of assistance in explaining the configuration of a blind hole and a counterbore;
Figs. 3(a) and 3(b) are diagrams of assistance in explaining the state of joining a wire to the blind hole formed in the suture needle;
Fig. 4 is a diagram of assistance in explaining the state of conducting a bending experiment of the wire joined to the suture needle; and
Fig. 5 is a diagram of assistance in explaining a problem, which is of assistance in explaining the state of performing a bending experiment of the metal wire of a suture needle with wire of the related art.

### Explanation of the Reference Numerals

- A: Suture needle
- 1: Body portion
- 2: Needle tip
- 3: Proximal end face
- 3a: Wall
- 4: Proximal end
- 5: Blind hole
- 6: Counterbore
- 10: Wire
- 11: Diameter reduction portion
- 12: Dent
- 13: Gap

### Best Mode for Carrying Out the Invention

The most preferred embodiments of a suture needle and a suture needle with wire according to the present invention will be described below. The present invention is the medical suture needle joined integrally to a wire used for suturing a bone, which improves the susceptibleness to bending damage of the wire.

The present inventors have considered the problem shown in Fig. 5 of the susceptibleness to bending damage of the wire of the suture needle joining the wire to a proximal end face, and have obtained the following findings. That is, in the suture needle, when the wire is inserted into a blind hole formed in the suture needle and is joined thereto by caulking, the entire portion inserted into the blind hole is constrained. When the suture needle is fixed to bend the wire to 90° with respect to the proximal end face, the wire is bent starting from the proximal end face and the largest bending stress acts on the vicinity of the proximal end face of the wire to cause the maximum extension. The wire is reversely bent so that the portion of the wire near the proximal end face is yielded to be bending damaged.

The present inventors have studied the problem repeatedly based on the findings, and have obtained directivity in which the wire is constrained so as to exhibit the appropriate degree of freedom by a slight distance without directly providing the degree of freedom of 180° bending to the wire from the end of the blind hole joined to the wire, whereby even if the wire is bent to 180° in appearance, its curvature is increased to reduce stress concentration, thereby enabling the reduction of the susceptibleness to bending damage.

The present inventors have conducted the experiment repeatedly based on the directionality, and have made the present invention. That is, in the suture needle of the present invention, the wire is inserted into the proximal end face for caulking to form the blind hole for joining the wire thereto, and the counterbore having a dimension at least in the proximal end face larger than a dimension of the blind hole and a depth smaller than a depth of a caulked portion with respect to the blind hole is formed on the proximal end face side of the blind hole.

In addition, in the suture needle with wire, the end of the wire is inserted into the blind hole formed in the proximal end face of the suture needle, and the outer circumferential portion of the suture needle corresponding to the blind hole is caulked to join the wire to the suture needle.

### [Embodiment 1]

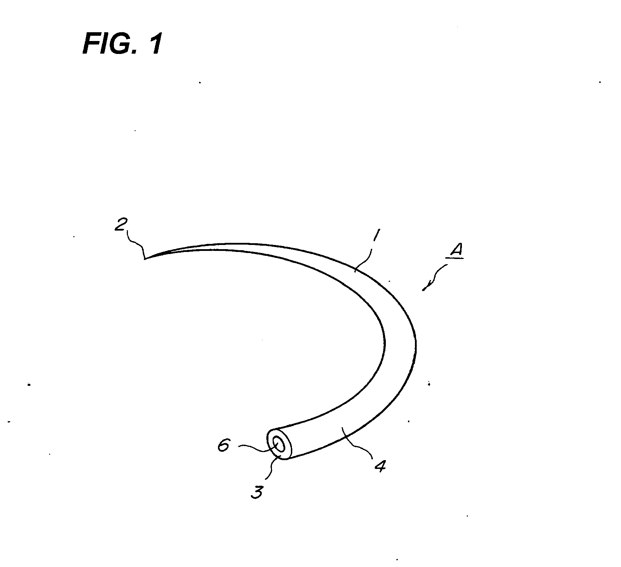

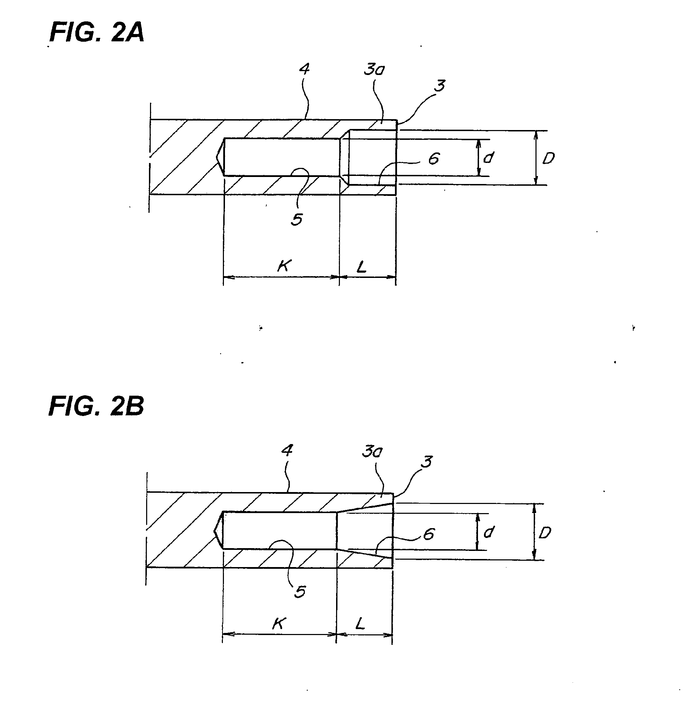

The configuration of the suture needle according to this embodiment will be described with reference to the drawings. Fig. 1 is a diagram of assistance in explaining the overall configuration of the suture needle. Figs. 2(a) and 2(b) are cross-sectional views of a proximal end portion of the suture needle, which are of assistance in explaining the configuration of the blind hole and the counterbore.

A medical suture needle A shown in Fig. 1 pierces through a bone to be sutured, and has a body portion 1 of circular cross section. The body portion 1 has one end formed with a needle tip 2 with a sharp pointed edge and the other end formed with a proximal end face 3. The proximal end face 3 side of the body portion 1 is formed as a proximal end 4.

In this embodiment, the cross section of the body portion 1 is circularly formed, but is not limited to the cross section shape, and may have a polygonal shape including a triangle in which any one of the edges is formed with a cutter. The needle tip 2 is not limited to a sharp pointed edge and may be obtuse so as to be formed in a spherical shape.

The material of the suture needle A is not limited and should secure a strength which can resist piercing resistance when piercing through the bone and a performance necessary for reducing piercing resistance. As such material, there are steel typified by carbon tool steel and stainless steel. These can be selectively used.

In particular, it is preferred to adopt austenitic stainless steel which can cause no rust in the distribution process to customers. The austenitic stainless steel cannot expect hardening by heat treatment. It is thus desirable to cold work and harden the austenitic stainless steel material.

As shown in Figs. 2(a) and 2(b), a blind hole 5 is formed in the depth direction from the proximal end face 3, and a counterbore 6 is formed on the proximal end face 3 side of the blind hole 5. The blind hole 5 has a dimension larger than the thickness dimension of a wire 10 to be joined and a depth which can sufficiently join the wire 10 thereto when the outer circumference portion of the blind hole 5 is caulked.

The blind hole 5 is formed so as to have a diameter which is about 0.02 mm larger than the thickness of the wire to be joined. When the thickness of the suture needle A is about 1.48 mm, the thickness of the wire is about 0.8 mm and the diameter of the blind hole 5 is set to about 0.82 mm. When the thickness of the suture needle A is about 1.58 mm, the thickness of the wire is about 1.0 mm and the diameter of the blind hole 5 is set to about 1.02 mm. The depth of the blind hole 5 is set to about 3.0 mm regardless of the dimension of the blind hole 5.

The method of forming the blind hole 5 is not limited and any of processingmethods such as drilling using a drill, electric discharge processing, and laser processing can be adopted. To ensure the shape accuracy and the dimensional accuracy of the blind hole 5, drilling is preferable.

The counterbore 6 is formed so as to have a dimension D at least in the proximal end face 3 larger than a dimension d of the blind hole 5 and a depth L smaller than a depth of a caulked portion K with respect to the blind hole 5. The counterbore 6 formed so that the dimension D at least in the proximal end face 3 is larger than the dimension d of the blind hole 5 may be a straight hole in the depth direction from the proximal end face 3, as shown in Fig. 2(a), and may be a tapered hole whose diameter is smaller in the depth direction from the proximal end face 3, as shown in Fig. 2(b). Various shapes such as a straight shape and a curved shape are applicable to the inner circumferential surface of the counterbore 6.

The method of forming the counterbore 6 in the proximal end face 3 is not limited. As such method of forming the counterbore 6, when the counterbore 6 is the straight hole, it is preferred to use a drill and a reamer. When the counterbore 6 is the tapered hole, it is preferred to use a cutter formed with a taper reamer and a tapered cutting edge.

As described above, the counterbore 6 should have the dimension D in the proximal end face 3 larger than the dimension d of the blind hole 5 and is not limited to the straight hole or the tapered hole. When the counterbore 6 is the tapered hole, the taper angle is not limited.

The difference between the dimension D of the counterbore 6 in the proximal end face 3 and the dimension d of the blind hole 5 becomes a dimension which can allow bending of the wire 10 joined to the blind hole 5 (a gap constraining the degree of freedom of the wire 10). It is preferred that the dimension difference be larger.

However, because the thickness of the suture needle A is fixed, the dimension D cannot be of course infinitely increased. When the dimension D is increased, the thickness of a wall 3a between the counterbore 6 and the outer circumference of the proximal end face 3 is reduced. The wall 3a can be affected and damaged by caulking with respect to the caulked portion K.

As described above, the value of the dimension D of the counterbore 6 in the proximal end face 3 is changed according to the conditions of the thickness of the proximal end 4 and the thickness of the wall 3a in the proximal end face 3 and cannot be uniquely set. For this reason, the suture needles with wire are manufactured as samples by joining the wire 10 to a plurality of suture needles A in which the dimension D and the depth L are changed. The bending damage experiment of the wire joined to each of the suture needles with wire is conducted to obtain data of the dimension D, the depth L, and the thickness of the wall 3a. A preferable range is set based on the data.

### [Embodiment 2]

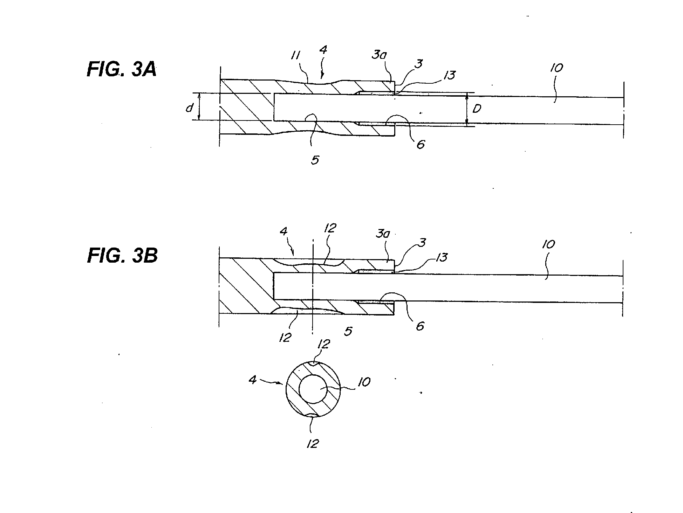

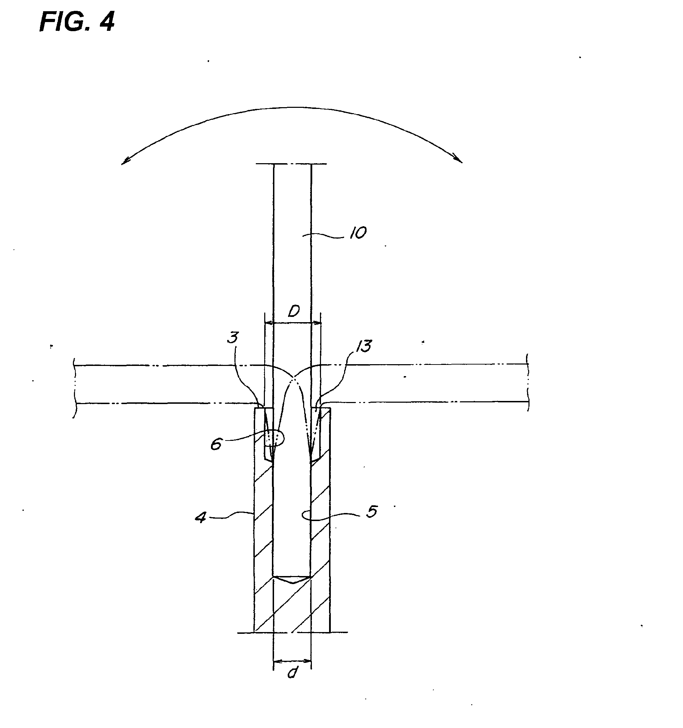

The experiment results will be described. Before the description, the configuration of the suture needle with wire according to this embodiment will be described with reference to the drawings. Figs. 3(a) and 3(b) are diagrams of assistance in explaining the state of joining the wire to the blind hole formed in the suture needle. Fig. 4 is a diagram of assistance in explaining the state of conducting a bending experiment of the wire joined to the suture needle. The same parts as the above embodiment in the drawings are indicated by similar reference numerals and the description is omitted.

In the suture needle with wire (medical suture needle) according to the present invention, the blind hole and the counterbore are formed in the proximal end face of the suture needle which can pierce through a bone and the metal wire 10 is joined integrally to the blind hole. In the present invention, it is essential that the blind hole and the counterbore be formed in the suture needle. The shapes and configurations of other portions are not limited. The configuration joining the wire to the suture needle will be described. In this embodiment, the suture needle A is used as the suture needle and the description of the configuration of the suture needle A is omitted.

In the suture needle with wire according to this embodiment, the end of the wire 10 is inserted into the blind hole 5 formed in the suture needle A, the portion of the proximal end 4 corresponding to the blind hole 5 is caulked, and the wire 10 is joined thereto to extend from the proximal end face 3.

The wire 10 uses a metal material and has a predetermined thickness and length. The thickness and length of the wire 10 are not limited and optimal dimensions are selected corresponding to the nature and portion of a bone to be sutured.

As the material of the wire 10, steel, stainless steel, and titanium can be selectively used. Above all, austenitic stainless steel which can cause no rust can be preferably used. Titanium which has excellent adaptability to a living body can be preferably used.

The reverse bending resistance performance of the wire using the titanium material tends to be slightly lower than the wire using the austenitic stainless steel material. Therefore, the suture needle with wire of the present invention can obtain a more preferable result when applied to the wire 10 using the titanium material.

The end of the wire 10 is inserted into the blind hole 5 formed in the suture needle A and is then joined thereto by caulking. The caulking operation is performed by pressing and deforming the outer circumferential portion of the proximal end 4 of the suture needle A corresponding to the blind hole 5. The method of the caulking operation is not limited. The wire 10 should be joined to the blind hole 5 so as not to be fallen out of the suture needle A at the time of a suturing operation.

As the caulking method of joining the wire 10 to the blind hole 5 formed in the suture needle A, there are a method of pressing the overall circumference of the portion of the proximal end 4 corresponding to the blind hole 5 to form a diameter reduction portion 11 for caulking, as shown in Fig. 3(a), a method of partially crushing the outer circumference of the proximal end 4 corresponding to the blind hole 5 to form a dent 12 for caulking, as shown in Fig. 3(b), and a method of forming the diameter reduction portion 11 over the overall circumference of the proximal end 4 and partially crushing the diameter reduction portion 11 to form the dent 12. These methods can be selectively adopted. The length dimension of the caulked portion K should be about 2/3 of the depth of the blind hole 5 (about 2.0 mm). As shown in the drawings, the outer circumference of the proximal end 4 corresponding to the counterbore 6 is not caulked so that the wire 10 can be hardly bending damaged.

When the wire 10 is joined to the blind hole 5, a ring-like gap 13 is formed between the outer circumferential surface of the wire 10 and the inner circumferential surface of the counterbore 6 in the proximal end face 3 of the suture needle A. The gap 13 secures the degree of freedom of movement of the wire 10 in the bending direction with respect to the suture needle A. The wire 10 can be freely moved within the angle range in which the outer circumference of the wire 10 is contacted with the wall 3a. When a portion of the outer circumference of the wire 10 is abutted on the wall 3a, the movement of the wire 10 is constrained.

Therefore, when the wire 10 is bent to 90° with respect to the proximal end face 3, the portion of the wire 10 corresponding to the counterbore 6 is held at an angle defined according to the depth L of the counterbore 6 and the dimension of the gap 13, starting from the portion of the wire 10 joined to the blind hole 5. Therefore, the wire 10 cannot be bent at a large curvature.

The wire 10 is bent to the proximal end face 3, starting from the portion of the wire 10 abutted on the wall 3a. The wire 10 has been already bent to the above angle in the portion of the wire 10 joined to the blind hole 5. The bending angle of 90° starting from the portion of the wire 10 abutted on the wall 3a becomes an angle to which the above angle is added. Thus, the wire 10 is bent to an angle larger than 90°. That is, the curvature of the wire 10 can be reduced.

Thus, the suture needle with wire according to the present invention can improve the susceptibleness to bending damage of the wire 10.

An experiment which measures the susceptibleness to bending damage of the wire 10 when the dimension D of the counterbore 6 in the proximal end face 3 and the depth L are changed will be described. In this experiment, the suture needle A to which the wire 10 is joined is fixed by a vice, the wire 10 is bent with respect to the proximal end face 3 to perform 180° reverse bending in a reciprocation, and the wire 10 which withstands three-time reverse bending is accepted.

The blind hole 5 having the dimension d of 0.82 mm and a depth of 3.0 mm is formed in the suture needle A having a thickness (outer diameter T) of 1.48 mm. The thickness of the wire 10 joined to the suture needle A is 0.8 mm.

In experiment 1, six samples formed with the counterbore 6 having the dimension D in the proximal end face 3 of 0.9 mm and the depth L of 0.5 mm are manufactured. In the samples, the thickness (t) of the wall 3a is 0.29 mm and the gap 13 has a dimension of 0.04 mm. In addition, d/D is 91.1%, t/T is 19.6%, and L/D is 55.6%.

In experiment 1, one of the six samples is bending damaged by two-time reverse bending and the remaining five samples can withstand three-time reverse bending. It is considered that they can be satisfactory as a product.

In experiment 2, six samples formed with the counterbore 6 having the dimension D in the proximal end face 3 of 1.0 mm and the depth L of 0.5 mm are manufactured. In the samples, the thickness (t) of the wall 3a is 0.24 mm and the gap 13 has a dimension of 0.09 mm. In addition, d/D is 82.0%, t/T is 16.2%, and L/D is 50.0%.

In experiment 2, all of the six samples can withstand three-time reverse bending. They are satisfactory as a product under the conditions of experiment 2.

In experiment 3, six samples formed with the counterbore 6 having the dimension D in the proximal end face 3 of 1.2 mm and the depth L of 0.2 mm are manufactured. In the samples, the thickness (t) of the wall 3a is 0.14 mm and the gap 13 has a dimension of 0.19 mm. In addition, d/D is 68.3%, t/T is 9.5%, and L/D is 16.7%.

In experiment 3, all of the six samples can withstand three-time reverse bending. They are satisfactory as a product under the conditions of experiment 3.

In experiment 4, six samples formed with the counterbore 6 having the dimension D in the proximal end face 3 of 1.3 mm and the depth L of 0.2 mm are manufactured. In the samples, the thickness (t) of the wall 3a is 0.09 mm and the gap 13 has a dimension of 0.24 mm. In addition, d/D is 63.1%, t/T is 6.1%, and L/D is 15.4%.

In experiment 4, caulking is failed in all of the six samples. It is considered that this is because the wall 3a is too thin.

In comparative experiment 1, six samples which are the suture needle A having a thickness of 1.48 mm, have the dimension d of 0.82 mm, and are not formed with the counterbore 6 are manufactured for experiment. All of the six samples are bending damaged by up to two-time reverse bending.

The blind hole 5 having the dimension d of 1.02 mm and a depth of 3.0 mm is formed in the suture needle A having a thickness (outer diameter T) of 1.58 mm. The thickness of the wire 10 joined to the suture needle A is 1.0 mm.

In experiment 5, six samples formed with the counterbore 6 having the dimension D in the proximal end face 3 of 1.2 mm and the-depth L of 0.5 mm are manufactured. In the samples, the thickness (t) of the wall 3a is 0.19 mm and the gap 13 has a dimension of 0. 09 mm. In addition, d/D is 85.0%, t/T is 8.9%, and L/D is 41.7%.

In experiment 5, all of the six samples can withstand three-time reverse bending. They are satisfactory as a product under the conditions of experiment 5.

In comparative experiment 2, six samples which are the suture needle A having a thickness of 1.58 mm and are not formed with the counterbore 6 are manufactured for experiment. All of the six samples are bending damaged by up to two-time reverse bending.

From the results of the experiments, it is preferred that the range of d/D be 65% to 95% and the range of t/T be 6.5% to 20%. The respective lower limits secure a sufficient wall thickness. The respective upper limits withstand reverse bending.

It is preferred that the range of L/D be 15.5% to 110%. The lower limit withstands reverse bending. For the upper limit, as described above, the caulked portion K should be about 2.0 mm at the minimum, L is about 1.0 mm at the maximum, and D is 0.9 mm at the maximum.

The suture needle A of the present invention can improve the susceptibleness to bending damage with respect to reverse bending without changing the material and thickness of the wire 10. Therefore, the suture needle A is advantageously used as a suture needle used for suturing bones.

## Claims

1. A medical suture needle which joins a metal wire to a proximal end face, comprising a blind hole which is formed in the proximal end face, inserts the end of the metal wire thereinto, and joins the metal wire thereto by caulking, and a counterbore which is formed on the proximal end face side of the blind hole and has a dimension at least in the proximal end face larger than a dimension of the blind hole and a depth smaller than a depth of a caulked portion with respect to the blind hole.

2. The medical suture needle,
wherein when the dimension of the counterbore in the proximal end face is D, the depth of the counterbore is L, the dimension of the blind hole is d, the outer diameter of the suture needle is T, and the thickness of a wall between the counterbore and the outer circumference of the proximal end face is t (t = T - D), the range of d/D is 65% to 95%, the range of t/T is 6.5% to 20%, and the range of L/D is 15.5% to 110%,
wherein a depth K of the caulked portion is 2.0 mm at the minimum, L is 1.0 mm at the maximum, and D is 0.9 mm at the maximum.

3. A medical suture needle with metal wire, comprising a blind hole which is formed in a proximal end face and inserts the end of a metal wire thereinto, and a counterbore which is formed on the proximal end face side of the blind hole and has a dimension at least in the proximal end face larger than a dimension of the blind hole, wherein the end of the metal wire is inserted into the blind hole to caulk the outer circumference of the medical suture needle corresponding to the blind hole, thereby integrating the metal wire therewith.

4. The medical suture needle with metal wire, wherein when the dimension of the counterbore in the proximal end face is D, the depth of the counterbore is L, the dimension of the blind hole is d, the outer diameter of the suture needle is T, and the thickness of a wall between the counterbore and the outer circumference of the proximal end face is t (t = T - D), the range of d/D is 65% to 95%, the range of t/T is 6.5% to 20%, and the range of L/D is 15.5% to 110%,
wherein a depth K of the caulked portion is 2.0 mm at the minimum, L is 1.0 mm at the maximum, and D is 0.9 mm at the maximum.
